# EUROPEAN PATENT SPECIFICATION

(11) **EP 1 458 355 B1**
(45) Date of publication and mention of the grant of the patent: **06.05.2009**
(21) Application number: 02799315.3
(22) Date of filing: 26.12.2002
(51) Int. Cl.: A61K 31/66, A61P 23/00

(54) **AQUEOUS BASED PHARMACEUTICAL FORMULATIONS OF WATER-SOLUBLE PRODRUGS OF PROPOFOL**
WÄSSRIGE PHARMAZEUTISCHE FORMULIERUNGEN VON WASSERLÖSLICHEN PRODRUGS VON PROPOFOL-VERBINDUNGEN
FORMULATIONS PHARMACEUTIQUES AQUEUSES DE PRODROGUES DE PROPOFOL SOLUBLES DANS L'EAU

(30) Priority: 28.12.2001 US 342755 P
(43) Date of publication of application: 22.09.2004
(73) Proprietor: Eisai Corporation of North America, Woodcliff Lake, NJ 07677 (US)
(72) Inventor: ROGERS, Tracey, L., Silver Spring, MD 20904 (US); WILLIAMSON, Jeffrey, A., Greenville, North Carolina 27834 (US); RHODES, Christopher, A., Ellicott City, MD 21042 (US)
(74) Representative: Bausch, Thorsten
(86) International application number: PCT/US2002/041468
(87) International publication number: WO 2003/057153

(56) References cited:
- WO-A-01/89514
- US-A- 5 908 869
- US-B1- 6 204 257
- SHAO X, LI H, WHITE P F, KLEIN K W, KULSTAD CH, OWENS A: "Bisulfite-containing propofol: Is it a cost-effective alternative to Diprivan for induction of anesthesia?" ANESTHESIA AND ANALGESIA, vol. 91, October 2000 (2000-10), pages 871-875, XP008043108
- FARINOTTI R: "INTERACTIONS PHYSICOCHIMIQUES ET MODE DE CONSERVATION DU DIPRIVAN PHYSICOCHEMICAL INTERACTIONS AND MODE OF STORAGE OF DIPRIVAN" ANNALES FRANCAISES D'ANESTHESIE ET DE REANIMATION, MASSON, PARIS, FR, vol. 13, no. 4, 1994, pages 453-456, XP009000415 ISSN: 0750-7658
- HAN J, DAVIS S S, WASHINGTON C: "Physical properties and stability of two emulsion formulations of propofol" INTERNATIONAL JOURNAL OF PHARMACEUTICS, vol. 215, 14 March 2001 (2001-03-14), pages 207-220, XP002318109
- HART B: "Diprivan: a change of formulation" EUROPEAN JOURNAL OF ANAESTHESIOLOGY, vol. 17, 2000, pages 71-73, XP002318110
- MANGIAMELI S, CALABRESE V, CANTARELLA G, MILAZZO G, NICOLOSI D, RIZZA V: "Compatibility of reduced glutathione (GSH) with different solutions currently used in anesthesia and reanimation" MINERVA ANESTESIOLOGICA, vol. 61, 1995, pages 407-409, XP008043093
- CRAIG D B: "Preservatives in propofol" CANADIAN JOURNAL OF ANESTHESIOLOGY, vol. 45, no. 9, 1998, page 913, XP008043094
- LEKAMP J E, DOBESH P P: "Propofol and too much sulfite?" CHEST, vol. 118, no. 1, July 2000 (2000-07), page 277, XP002318111
- BAKER M T: "Yellowing of metabisulfite-containing propofol emulsion" AMERICAN JOURNAL OF HEALTH-SYSTEM PHARMACY, vol. 58, 1 June 2001 (2001-06-01), pages 1042-1047, XP008043095

## Description

### FIELD OF THE INVENTION

The present invention is directed to aqueous based formulations of water-soluble prodrugs of propofol.

### BACKGROUND OF THE INVENTION

The successful delivery of a pharmaceutical to a patient is of critical importance in the treatment of disorders. However, the use of many clinical drugs with known bioactive properties is limited by the drugs' very low water solubility. As a result of low water solubility, many drugs often are formulated in co-solvent pharmaceutical vehicles, including surfactants. Such surfactants have been shown to lead to severe side effects in humans that limit the clinical safety of these drugs and therefore the treatment of several disorders.

Propofol (2,6-diisopropylphenol or DIP) is a low molecular weight phenol derivative that is widely used as a hypnotic or sedative agent for intravenous administration in the induction and maintenance of anesthesia or sedation in humans and animals. Among its useful characteristics as an anesthetic drug are: administration via the intravenous route, rapid onset and offset of anesthesia, rapid clearance, and a side-effect profile that makes it preferable to other injectable anesthetics, such as barbiturates.

The use of injectable anesthetic agents generally, and of propofol specifically, in the induction and maintenance of general anesthesia has gained widespread acceptance in anesthetic care over the last 15 years. Intravenous anesthesia with propofol has been described to have several advantages over preexisting methods, such as more readily tolerated induction, since patients need have no fear of masks, suffocation, or the overpowering smell of volatile anesthetics; rapid and predictable recovery; readily adjustable depth of anesthesia by adjusting the IV dose of propofol; a lower incidence of adverse reactions as compared to inhalation anesthetics; and decreased dysphoria, nausea, and vomiting upon recovery from anesthesia (Padfield N.L., Introduction, History and Development, in: Padfield NL (Ed.) Ed., Total Intravenous Anesthesia, Butterworth Heinemann, Oxford 2000).

In addition to its sedative and anesthetic effects, propofol has a range of other biological and medical applications. For example, it has been reported to be an anti-emetic (McCollum JSC et al., Anesthesia 43 (1988) 239), an anti-epileptic (Chilvers C.R., Laurie P.S., Anesthesia 45 (1990) 995), and an anti-pruritic (Borgeat et al., Anesthesiology 76 (1992) 510). Anti-emetic and anti-pruritic effects are typically observed at sub-hypnotic doses, i.e. at doses that achieve propofol plasma concentrations lower than those required for sedation or anesthesia. Anti-epileptic activity, on the other hand, is observed over a wider range of plasma concentrations (Borgeat et al., Anesthesiology 80 (1994) 642). It has further been speculated that propofol, due to its antioxidant properties in biological systems, may be useful in the treatment of inflammatory conditions, especially inflammatory conditions with a respiratory component, and in the treatment of neuronal damage related to neurodegeneration or trauma. Such conditions are believed to be associated with the generation of reactive oxygen species and therefore amenable to treatment with antioxidants (see, e.g. U.S. Patent 6,254,853 to Hendler et al.)

Propofol typically is formulated for clinical use as a oil-in-water emulsion. The formulation has a limited shelf-life and has been shown to be sensitive to bacterial or fungal contamination, which has led to instances of post-surgical infections (Bennett S.N. et al., N Eng. J Med 333 (1995) 147). Due to the dense, white color of the formulation, bacterial or fungal contamination cannot be detected by visual inspection of the vial in the first instance.

Not only is propofol poorly water soluble, but it also causes pain at the injection site, which must often be alleviated by using a local anesthetic (Dolin S.J., Drugs and Pharmacology, in: N. Padfield, Ed., Total Intravenous Anesthesia, Butterworth Heinemann, Oxford 2000). Due to its formulation in a lipid emulsion, its intravenous administration is also associated with undesirable hypertriglyceridemia in patients, especially in patients receiving prolonged infusions (Fulton B. and Sorkin E.M., Drugs 50 (1995) 636). Its formulation as a lipid emulsion further makes it difficult to co-administer other IV drugs. Any physical changes to the formulation, such as a change in lipid droplet size, can lead to changes in the pharmacological properties of the drug and cause side effects, such as lung embolisms.

It has further been reported that the use of propofol in anesthesia induction is associated with a significant incidence of apnea, which appears to be dependent on dose, rate of injection, and pre-medication (Reves, J.G., Glass, P.S.A., Lubarsky D.A., Non-barbiturate Intravenous Anesthetics. In: R.D. Miller et al., Eds, Anesthesia. 5th Ed. Churchill Livingstone, Philadelphia, 2000). Respiratory consequences of administering anesthetic induction doses of propofol, including a reduction in tidal volume and apnea, occur in up to 83% of patients (Bryson et al., Drugs 50 (1995) at 520). Induction doses of propofol are also known to have a marked hypotensive effect, which is dose- and plasma concentration-dependent (Reves et al., *supra*). The hypotension associated with peak plasma levels after rapid bolus injection of propofol sometimes requires the use of controlled infusion pumps or the breaking-up of the induction bolus dose into several smaller incremental doses. Further, the short duration of unconsciousness caused by bolus induction doses renders propofol suitable for only brief medical procedures. For all the above reasons, propofol for induction and/or maintenance of anesthesia must normally be administered in an in-patient setting under the supervision of an anesthesiologist, and is often considered inappropriate for use by non-anesthesiologists in an ambulatory or day case setting.

In addition to its use in induction and maintenance of anesthesia, propofol has been used successfully as a sedative to accompany either local or regional anesthesia in conscious patients. Its sedative properties have also been exploited in diagnostic procedures that have an unsettling effect on conscious patients, such as colonoscopy or imaging procedures. Propofol has also been used as a sedative in children undergoing diagnostic imaging procedures or radiotherapy. A recent development is that of patient-controlled sedation with propofol. This technique is preferred by patients and is as effective as anesthesiologist-administered sedation.

Compared with the widely used sedative midazolam or other such agents, propofol provided similar or better sedative effects when the quality of sedation and/or the amount of time that patients were at adequate levels of sedation were measured (*see* Fulton B. and Sorkin E.M., Drugs 50 (1995) 636). The faster recovery and similar or less amnesia associated with propofol makes it an attractive alternative to other sedatives, particularly for patients requiring only short sedation. However, because of the potential for hyperlipidemia associated with the current propofol formulation, and the development of tolerance to its sedative effects, the usefulness of propofol for patients requiring longer sedation is less well established. For all the reasons given above, there exists a clinical need for aqueous, stable formulations of safe, injectable, or infusible sedative or hypnotic agents.

The development of water soluble and stable prodrugs of propofol, which is described in U.S. Patent 6,204,257 to Stella et al., has made it possible to address these heretofore unmet needs, and to explore the pharmaceutical advantages of an aqueous propofol-prodrug in the induction and maintenance of sedation and anesthesia in patients. The prodrugs of the present invention differ from propofol in that the 1-hydroxy-group of propofol is replaced with a phosphonooxymethyl ether group: 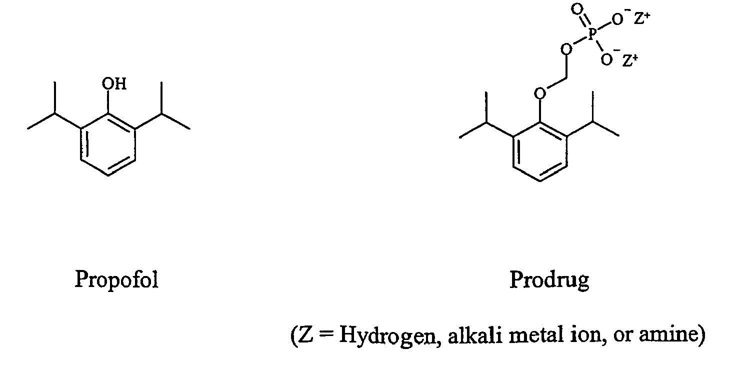 While not wanting to be bound by theory, the prodrug is believed to undergo hydrolysis by endothelial cell surface alkaline phosphatases to release propofol.

Propofol currently is formulated as an oil-in-water emulsion. For example, U.S. Patent 6,177,477 to George et aL describes a sterile pharmaceutical composition of propofol for parenteral administration comprising an oil-in-water emulsion in which propofol is dissolved in a water-immiscible solvent and is emulsified with water containing tromethamine as a preservative. The preservative is said to be present in an amount sufficient to prevent any significant growth of microorganism for at least 24 hours in the event of extrinsic contamination. Because propofol is formulated as an emulsion, it is difficult and questionable to add other drugs to the formulation, as physical changes to the formulation, such as an increase in oil droplet size, can lead to lung embolisms or other complications.
WO 01/89514 A2 relates to a method of treating and improving survival in critically ill patients and the use of certain sterile sedative pharmaceutical compositions for the manufacture of a medicament for improving the survival of said patients. The pharmaceutical composition according to WO 01/89514 may contain a metal iron chelating agent such as EDTA.
Xinli Shao et al., Anesth. Analg. 2000, vol. 91, pages 871- 875, relates to bisulfite containing propofol. The propofol formulations according to this article contain an anti-microbial agent such as sodium metabisulfite.
R. Farinotti et al., Ann Fr. Anesth. Réanim., vol. 13, 1994, pages 453 - 556, describes physio-chemical interaction and storage forms of Diprivan (propofol). In this article the dimer and quinone formation of propofol in the presence of oxygen is described.
J. Han et al., International Journal of Pharmaceutics, vol. 215, 2001, pages 207 - 220, is directed to physical properties and stability of two emulsion formulations of propofol. The described emulsions contain either disodium EDTA or sodium metabisulfite for stabilization.
B. Hart, European Journal of Anaesthesiology, vol. 17, 2000, pages 71 - 73, describes modified formulations of Diprivan in response to the observation of a number of cases of infections in patients as a result of inappropriate handling of Diprivan by healthcare professionals. The modified formulation of Diprivan contains disodium EDTA.
S. Mangiameli et al., Minerva Anestesiol., vol. 61. 1995, pages 407 - 409, is directed to the compatibility of reduced glutathione (GSH) with different solutions currently used in anaesthesia and reanimation.
Douglas B. Craig, Can. J. Anaesth., 1998, pages 913 -916, relates to preservatives in propofol. The use of disodium EDTA in propofol is critically discussed.
Jonathan E. Lekamp et al., Chest, vol. 118, page 277, 2000, is concerned with propofol and the sulfite content contained therein which origins from sodium metabisulfite.
Max T. Baker, Am. J. Health-Syst. Pharm., 2001, vol. 48, pages 1044 - 1045, is concerned with yellowing of metasulfite containing propofol emulsion.
US 6,204,257 By relates to novel water-soluble prodrugs of aliphatic or aromatic hindered hydroxyl group containing pharmaceuticals *inter alia* comprising propofol as well as prodrugs thereof.

It would be desirable to develop a room temperature-stable, aqueous-based formulation for water-soluble prodrugs of propofol, especially a formulation that does not require potentially toxic co-solvents or the use of surfactant.

### BRIEF SUMMARY OF THE INVENTION

The present invention is directed to aqueous based pharmaceutical formulations of water-soluble prodrugs of propofol. The pharmaceutical formulation comprises in an aqueous medium a therapeutically effective amount of a compound represented by Formula I: 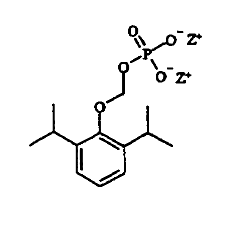 wherein each Z is independently selected from the group consisting of hydrogen, alkali metal ion, and amine, and an effective amount of an antioxidant. The pharmaceutical formulation may also contain other components, such as a tonicity modifier and/or a buffer.

According to another embodiment of the present invention, an aqueous-based formulation comprises an effective amount of a compound of Formula I, an antioxidant, and optionally a buffer. The amount of the compound of Formula I present in the formulation is such that the tonicity, *i.e.,* osmolality, is essentially the same as normal physiological fluids.

Preferred formulations of the present invention are particularly useful as intravenous injections. The formulations preferably are buffered to a pH suitable for minimizing degradation of the prodrug during storage. The formulations can be prepared without the use of harmful co-solvents or surfactants, and are stable at room temperature over extended periods of time.

### DETAILED DESCRIPTION OF THE INVENTION

The pharmaceutical formulations of the present invention comprise in aqueous medium, a therapeutically effective amount of a water-soluble prodrug represented by Formula I: 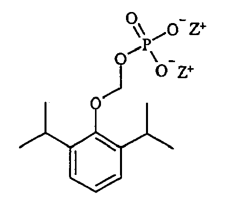 wherein each Z is independently selected from the group consisting of hydrogen, alkali metal ion, and amine, and an effective amount of an antioxidant. The aqueous-based formulations may also contain other components, such as a tonicity modifier and/or a buffer.

Methods for synthesizing the derivatives of Formula I are described in U.S. Patent 6,204,257 By, the disclosure of which hereby is incorporated by reference in its entirety. A representative example of a compound of Formula I is O-phosphanooxymethyl-propofol, the structure of which is illustrated below:

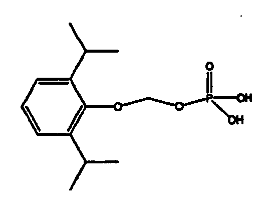

The relative amount of the prodrug in the formulation can vary over a wide range depending on a variety of factors including but not limited to the identity of the prodrug, the bioactivity of the parent drug for a particular disorder being treated, and the intended mode of administration. The relative amount of the prodrug in the formulation most often ranges from 0.5 to 20% (w/v), more usually from 1 to 10%.

Any pharmaceutically acceptable aqueous medium, such as water of sufficiently high purity, may be used in the formulations of the present invention.

The antioxidant prevents or reduces oxidative degradation of the prodrug into poorly water-soluble compounds. The prodrug is believed to be converted to DIP by aqueous hydrolysis or by enzymatic processes in the blood. DIP in turn is converted to the related substances quinone and hydroquinone by an oxidative process. All three of DIP, quinone, and hydroquinone are poorly water-soluble. It is desirable to minimize the formation or presence of poorly water-soluble compounds in the aqueous-based formulation because even at low concentrations, these compounds impart a yellow color to the solution. Over time, the solution becomes hazy, and eventually particles form.

The antioxidant should be present in at least a minimum amount that provides some reduction in oxidative degradation of the water-soluble prodrug. There is no particular maximum concentration contemplated. The concentration of the antioxidant in the aqueous formulation most often ranges from 0.1 to 1% (w/v). During manufacturing, the solution may be sparged with nitrogen to reduce the level of dissolved oxygen present in the formulation, which also provides protection against oxidative degradation.

A variety of antioxidants may be used in the formulations of the present invention. The particular antioxidant used can be suitably selected in accordance with such factors as the particular prodrug(s) present in the formulation. Non-limiting examples of antioxidants include monothioglycerol, glutathione, citric acid, ascorbic acid, sodium metabisulfite, and sodium sulfite. EDTA, a metal chelator, provides protection from catalytic oxidation of phenols.

Because preferred formulations are intended for parenteral administration, it is preferable to make up solutions such that the tonicity, *i.e*., osmolality, is essentially the same as normal physiological fluids in order to prevent post-administration swelling or rapid absorption of the composition because of differential ion concentrations between the composition and physiological fluids. If needed, a tonicity modifier is present in a suitable amount that can be ascertained by persons skilled in the art with the aid of no more than routine experimentation. When used, the amount of tonicity modifier used most often ranges from 0.1 to 1% (w/v). The particular tonicity modifier used is not critical to the practice of the present invention. Non-limiting examples of suitable tonicity modifiers include sodium chloride, glycerin, boric acid, calcium chloride, dextrose, and potassium chloride.

The pH of the formulation preferably is maintained to provide long-term stability of the formulation at room temperature. In most cases a suitable pH is from 7 to 10, and preferably is at least 8.5. The solution may be buffered using any standard buffer effective in the pH range of 7-10, *e.g*., carbonate, phosphate, borabe, or glycine. One preferred buffer is tromethamine (2-amino-2-hydroxymethyl-1,3-propanediol), also commonly referred to as TRIS. The amount of buffer needed for this purpose most often ranges from 10 to 25 mmol.

Other components may be present in the formulation. For example, in the case of a multi-dose vial, a preservative may be included, such as benzyl alcohoL The formulation also may contain co-solvents such as polyethylene glycol (PEG 200, PEG 400), propylene glycol, and/or ethanol. Concentrations of the co-solvents can vary over a wide range, most often from 0 to 20%.

The formulations of the present invention may be administered via any suitable route of administration. Formulations for intravenous injection may be packaged, for example, in a glass vial, in a pre-filled syringe, or in an ampoule. The formulations may be administered with standard IV diluent solutions, *e.g.,* D5W, normal saline, or Lactated Ringer's solution.

Suitable dosages can be ascertained depending on such factors as the identity of the prodrug and the type of the disorder being treated. Dosages may be, for example, in the range of 0.1 to 100 mg/kg of body weight, or 5 to 500 mg/ml. As will be apparent to persons skilled in the art, many factors that modify the action of the drug will be taken into account in determining the dosage including the age, sex, diet and physical conditions of the patient.

For administering the propofol prodrug, an anesthesiologist skilled in the art of anesthesia will be able to ascertain, without undue experimentation, an appropriate treatment protocol for administering a formulation of the present invention. The dosage, mode and schedule of administration are not particularly restricted, and will vary with the particular indication. The formulation may be administered parenterally. The dosage may be, for example, in the range of 0.5 to 10 mg/kg administered according to procedures for induction of general anesthesia or maintenance of general anesthesia. Alternatively, the formulation may be administered by parenteral infusion, the dosage may be, for example, in the range of 2 µg/kg/min to 800 µg/kg/min administered according to procedures for maintenance of general anesthesia, initiation and maintenance of MAC sedation or initiation and maintenance of ICU sedation.

### EXAMPLES

The following examples are provided to illustrate the present invention.

### Example 1

This example illustrates preparing a 2% solution of O-phosphonooxymethyl propofol, a water-soluble prodrug of propofol. The aqueous-based formulation has the composition set forth in Table 1 below.

**Table 1**

| **Component** | **Concentration** |
|---|---|
| O-phosphonooxymethyl propofol | 2% (20 mg/ml) |
| Sodium Chloride | 0.4% |
| Monothioglycerol | 0.5% |
| TRIS, USP (Tromethamine) | 20 mmol |
| pH | 9±0.5 |

Sodium chloride (28 g) was added to 7 1 of water and stirred until dissolved. Next, TRIS (2-amino-2-hydroxymethyl-1,3-propanediol) (20 mmol) was added with stirring. The solution was then sparged with nitrogen gas. Monothioglycerol (35 g) then was added with stirring. O-phosphonooxymethyl propofol (140 g) was added, and the solution was stirred until it dissolved. The solution was filtered and poured into vials.

### Example 2

This example illustrates preparing a 4% solution of O-phosphonooxymethyl propofol. The aqueous-based formulation has the composition set forth in Table 2 below.

**Table 2**

| **Component** | **Concentration** |
|---|---|
| O-phosphonooxymethyl propofol | 4% (40 mg/ml) |
| Monothioglycerol | 0.25% |
| TRIS, USP (Tromethamine) | 20 mmol |
| pH | 9±0.5 |

TRIS (20 mmol) was added to 71 of water with stirring. The solution was then sparged with nitrogen gas. Monothioglycerol (17.5 g) then was added with stirring. O-phosphonooxymethyl propofol (280 g) was added, and the solution was stirred until it dissolved. The solution was filtered and poured into vials.

### Example 3

This example illustrates preparing a 2% solution of O-phosphonooxymethyl propofol having the composition set forth in Table 3 below.

**Table 3**

| **Component** | **Concentration** |
|---|---|
| O-phosphonooxymethyl propofol | 2% (20 mg/ml) |
| Sodium Chloride | 0.4% |
| Monothioglycerol | 0.5% |
| Carbonate buffer | 20 mmol |
| pH | 9±0.5 |

Sodium chloride (28 g) is added to 7 1 of water and stirred until dissolved. Next, carbonate buffer (20 mmol) was added with stirring. The solution was then sparged with nitrogen gas. Monothioglycerol (35 g) then was added with stirring. O-phosphonooxymethyl propofol (140 g) was added, and the solution was stirred until it dissolved. The solution was filtered and poured into vials.

### Example 4

This example illustrates preparing an unbuffered 2% solution of O-phosphonooxymethyl propofol having the composition set forth in Table 4 below.

**Table 4**

| **Component** | **Concentration** |
|---|---|
| O-phosphonooxymethyl propofol | 2% (20 mg/ml) |
| Sodium Chloride | 0.4% |
| Monothioglycerol | 0.5% |
| pH | 9±0.5 |

Sodium chloride (28 g) was added to 71 of water and stirred until dissolved. The solution is then sparged with nitrogen gas. Monothioglycerol (35 g) then was added with stirring. O-phosphonooxymethyl propofol (140 g) was added, and the solution was stirred until it dissolved. The solution was filtered and poured into vials.

### Example 5

This example illustrates that a 2% solution of O-phosphonooxymethyl propofol can be prepared using 0.1% sodium sulfite as an antioxidant. The aqueous-based formulation has the composition set forth in Table 5 below.

**Table 5**

| **Component** | **Concentration** |
|---|---|
| O-phosphonooxymethyl propofol | 2% (20 mg/ml) |
| Sodium Chloride | 0.4% |
| Sodium Sulfite | 0.1% |
| TRIS, USP (Tromethamine) | 20 mmol |
| pH | 9±0.5 |

Sodium chloride (28 g) is added to 7 1 of water and is stirred until it dissolves. Next, TRIS (2-amino-2-hydroxymethyl-1,3-propanediol) (20 mmol) is added with stirring. The solution is then sparged with nitrogen gas. Sodium sulfite (7 g) then is added with stirring. O-phosphonooxymethyl propofol (140 g) is added, and the solution is stirred until it dissolves. The solution is filtered and poured into vials.

## Claims

1. A pharmaceutical formulation comprising in an aqueous medium:
(i) a therapeutically effective amount of a compound represented by the formula 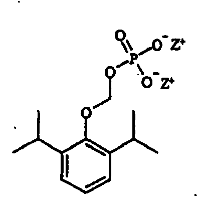 wherein each Z is independently selected from the group consisting of hydrogen, alkali metal ion, and amine; and
(ii) an effective amount of an antioxidant.

2. The formulation of claim 1 wherein the antioxidant is selected from the group consisting of monothioglycerol, glutathione, citric acid, ascorbic acid, sodium metabisulfite, sodium sulfite, and EDTA.

3. The formulation of claim 2 wherein the concentration of the antioxidant is from 0.1 to 1% (w/v).

4. The formulation of claim 1 further comprising a tonicity modifier.

5. The formulation of claim 4 wherein the tonicity modifier is selected from the group consisting of sodium chloride, glycerin, boric acid, calcium chloride, dextrose, and potassium chloride.

6. The formulation of claim 5 wherein the concentration of the tonicity modifier is from 0.1 to 1% (w/v).

7. The formulation of claim 1 further comprising a buffer.

8. The formulation of claim 7 wherein the buffer is 2-amino-2-hydroxymethyl-1,3-propanediol.

9. A pharmaceutical formulation comprising in an aqueous medium:
(i) a therapeutically effective amount of O-phosphonooxymethyl propofol;
(ii) from about 0.1 to 1% (w/v) of an antioxidant selected from the group consisting of monothioglycerol, glutathione, citric acid, ascorbic acid, sodium metabisulfite, sodium sulfite, and EDTA; and
(iii) from 0.1 to 1% (w/v) of a tonicity modifier selected from the group consisting of sodium chloride, glycerin, boric acid, calcium chloride, dextrose, and potassium chloride.

## Patentansprüche

1. Pharmazeutische Formulierung, umfassend in einem wäßrigen Medium:
(i) eine therapeutisch wirksame Menge einer Verbindung der Formel: 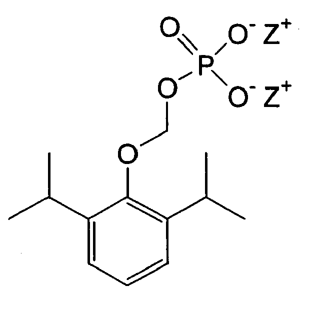 worin jedes Z unabhängig ausgewählt ist aus der Gruppe bestehend aus Wasserstoff, Alkalimetallionen und Aminen; und
(ii) eine wirksame Menge eines Antioxidationsmittels.

2. Formulierung gemäß Anspruch 1, worin das Antioxidationsmittel ausgewählt ist aus der Gruppe bestehend aus Monothioglycerol, Glutathion, Zitronensäure, Ascorbinsäure, Natriummetabisulfit, Natriumsulfit und EDTA.

3. Formulierung gemäß Anspruch 2, worin die Konzentration des Antioxidationsmittels von 0,1 bis 1% (G/V) beträgt.

4. Formulierung gemäß Anspruch 1, ferner umfassend einen Tonizitätsmodifizierer.

5. Formulierung gemäß Anspruch 4, worin der Tonizitätsmodifizierer ausgewählt ist aus der Gruppe bestehend aus Natriumchlorid, Glycerin, Borsäure, Calciumchlorid, Dextrose und Kaliumchlorid.

6. Formulierung gemäß Anspruch 5, worin die Konzentration des Tonizitätsmodifizierers von 0,1 bis 1 % (G/V) beträgt.

7. Formulierung gemäß Anspruch 1, ferner umfassend einen Puffer.

8. Formulierung gemäß Anspruch 7, worin der Puffer 2-Amino-2-hydroxymethyl-1,3-propandiol ist.

9. Pharmazeutische Formulierung, umfassend in einem wäßrigen Medium:
(i) eine therapeutisch wirksame Menge von O-Phosphonooxymethylpropofol;
(ii) von 0,1 bis 1% (G/V) eines Antioxidationsmittels, ausgewählt aus der Gruppe bestehend aus Monothioglycerol, Glutathion, Zitronensäure, Ascorbinsäure, Natriummetabisulfit, Natriumsulfit und EDTA; und
(iii) von 0,1 bis 1 % (G/V) eines Tonizitätsmodifizierers, ausgewählt aus der Gruppe bestehend aus Natriumchlorid, Glycerin, Borsäure, Calciumchlorid, Dextrose und Kaliumchlorid.

## Revendications

1. Formulation pharmaceutique comprenant dans un milieu aqueux :
(i) une quantité thérapeutiquement efficace d'un composé représenté par la formule 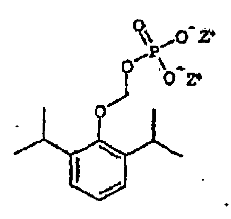 dans laquelle chaque Z est indépendamment choisi parmi le groupe consistant en l'hydrogène, un ion d'un métal alcalin, et une amine ; et
(ii) une quantité efficace d'un antioxydant.

2. Formulation selon la revendication 1 dans laquelle l'antioxydant est choisi parmi le groupe consistant en le monothioglycérol, le glutathion, l'acide citrique, l'acide ascorbique, le métabisulfite de sodium, le sulfite de sodium, et l'EDTA.

3. Formulation selon la revendication 2 dans laquelle la concentration de l'antioxydant est de 0,1 à 1% (en poids par volume).

4. Formulation selon la revendication 1 comprenant en outre un modificateur de la tonicité.

5. Formulation selon la revendication 4 dans laquelle le modificateur de la tonicité est choisi parmi le groupe consistant en le chlorure de sodium, la glycérine, l'acide borique, le chlorure de calcium, le dextrose, et le chlorure de potassium.

6. Formulation selon la revendication 5 dans laquelle la concentration du modificateur de la tonicité est de 0,1 à 1% (en poids par volume).

7. Formulation selon la revendication 1 comprenant en outre un tampon.

8. Formulation selon la revendication 7 dans laquelle le tampon est le 2-amino-2-hydroxyméthyl-1,3-propanediol.

9. Formulation pharmaceutique comprenant dans un milieu laqueux :
(i) une quantité thérapeutiquement efficace de O-phosphonooxyméthyle propofol ;
(ii) de 0,1 à 1% (en poids par volume) d'un antioxydant choisi parmi le groupe consistant en le monothioglycérol, le glutathion, l'acide citrique, l'acide ascorbique, le métabisulfite de sodium, le sulfite de sodium, et l'EDTA ; et
(iii) de 0,1 à 1% (en poids par volume) d'un modificateur de la tonicité choisi parmi le groupe consistant en le chlorure de sodium, la glycérine, l'acide borique, le chlorure de calcium, le dextrose, et le chlorure de potassium.
